# FASCICULE DE BREVET EUROPEEN

(11) **EP 4 408 558 B1**
(45) Date de publication et mention de la délivrance du brevet: **06.05.2026**
(21) Numéro de dépôt: 22789240.3
(22) Date de dépôt: 30.09.2022
(51) Int. Cl.: B01D 3/14, B01D 3/34, C07C 7/05, F25J 3/02

(54) **PROCÉDÉ DE SEPARATION DE TOUT OU PARTIE DES COMPOSES D'UN BIOGAZ A L'ETAT LIQUIDE OU A L'ETAT DIPHASIQUE**
VERFAHREN ZUR ABTRENNUNG ALLER ODER EINIGER VERBINDUNGEN AUS EINEM BIOGAS IN FLÜSSIGEM ZUSTAND ODER IN ZWEIPHASIGEM ZUSTAND
METHOD FOR SEPARATING ALL OR SOME OF THE COMPOUNDS FROM A BIOGAS IN THE LIQUID STATE OR IN THE TWO-PHASE STATE

(30) Priorité: 01.10.2021 FR 2110431; 08.11.2021 FR 2111835
(43) Date de publication de la demande: 07.08.2024
(73) Titulaire: Sublime Energie, 75009 Paris (FR)
(72) Inventeur: NAIT SAIDI, Chourouk, 91120 PALAISEAU (FR); RIVERA TINOCO, Rodrigo, 5914 PV VENLO (NL)
(74) Mandataire: IP Trust
(86) Numéro de dépôt international: PCT/EP2022/077360
(87) Numéro de publication internationale: WO 2023/052624

(56) Documents cités:
- FR-A1- 2 296 607
- FR-A1- 2 312 001
- US-A- 4 318 723
- US-A1- 2003 047 492

## Description

### DOMAINE TECHNIQUE DE L'INVENTION

La présente invention concerne le domaine de la valorisation des biogaz, et se rapporte plus particulièrement à un procédé de séparation de tout ou partie des composés d'un biogaz à l'état liquide ou à l'état diphasique liquide/vapeur, le biogaz contenant du méthane, du dioxyde de carbone et éventuellement des hydrocarbures de la famille C₃ à C₇permettant sa liquéfaction.

Le procédé selon l'invention est destiné notamment, mais non exclusivement, à l'épuration du biogaz à l'état liquide ou à l'état diphasique liquide/vapeur aux fins de récupérer tout ou partie de ses composés, et en particulier le méthane pour être utilisé comme carburant pour les véhicules ou pour être injecté dans un réseau de gaz naturel ainsi que le dioxyde de carbone.

### ETAT DE LA TECHNIQUE

Parmi les méthodes d'épuration du biogaz connues, les méthodes conventionnellement utilisées pour séparer le méthane du dioxyde de carbone sont la séparation membranaire, la PSA (Pressure Swing Adsorption - adsorption par variation de pression) et la distillation cryogénique.

La séparation membranaire est basée sur l'utilisation de membranes réalisées dans des matériaux laissant passer le dioxyde de carbone et retenant le méthane. La PSA est basée sur l'adsorption du dioxyde de carbone dans les composants solides de la colonne d'adsorption à haute pression et une désorption à basse pression. La distillation cryogénique repose quant à elle sur des abaissements successifs de la température jusqu'à la production de dioxyde de carbone liquide et de biométhane liquéfié ou de désublimation du dioxyde de carbone qui produit du dioxyde de carbone liquide et du bio-méthane gazeux.

Ces méthodes conventionnellement utilisées présentent cependant des inconvénients pour séparer un biogaz à l'état liquide ou à l'état diphasique. Soit elles ne permettent pas d'atteindre des taux de pureté du méthane récupéré satisfaisants, soit elles ne permettent pas de récupérer le dioxyde de carbone, soit elles s'avèrent inadaptées pour le traitement d'un biogaz liquéfié ou contenant des hydrocarbures, notamment le butane, utilisés pour liquéfier le biogaz. Ainsi la technique de la séparation membranaire n'est pas à privilégier lorsque le biogaz comporte des hydrocarbures, et en particulier du butane, ce dernier détériorant les membranes rapidement. La PSA ne permet pas d'atteindre quant à elle les taux de pureté souhaités. Par ailleurs, cette méthode nécessite l'apport d'une quantité importante de chaleur ce qui va à l'encontre de l'utilisation de froid préalablement dépensé pour liquéfier le biogaz. La technique de distillation cryogénique présente quant à elle l'inconvénient de produire des particules solides de dioxyde de carbone (cristaux de neige carbonique) qui peuvent créer des bouchons dans les échangeurs de chaleur et le garnissage dans la tête de colonne.

On connait également des demandes de brevet US4318723 et US2003/0047492 des procédés de séparation de composés. Cependant, les procédés décrits concernent la séparation des composés d'un gaz naturel, et ne s'adressent pas à la problématique de séparation des composés d'un biogaz et de récupération de chacun de ces composés à des taux de pureté de l'ordre de 99,9%.

L'invention vise à remédier à ces problèmes en proposant un procédé de séparation permettant de séparer les composés d'un biogaz à l'état liquide ou à l'état diphasique liquide/vapeur de manière à récupérer le méthane à un taux de pureté élevé ainsi que de récupérer le dioxyde de carbone en évitant les problèmes de colmatage et formation de neige carbonique.

### OBJET DE L'INVENTION

A cet effet, l'invention propose un procédé de séparation de la revendication 1.

L'injection d'un agent de liquéfaction en tête de colonne au-dessus de l'entrée du biogaz à une température et quantité déterminées, à savoir à une température inférieure ou égale à la température à laquelle le dioxyde de carbone dé-sublimerait dans la colonne de distillation maintenue à une pression donnée d'une part et en quantité proportionnelle au débit de vapeur du dioxyde de carbone ascendant en tête de colonne d'autre part, permet ainsi de dissoudre les cristaux de neige carbonique qui se forment en tête de la colonne de distillation utilisée pour séparer le méthane des autres composés en assurant une séparation satisfaisante du méthane gazeux du mélange liquide composé du dioxyde de carbone et de l'agent de liquéfaction.

Par température de désublimation, on désigne la température à laquelle la désublimation (passage de l'état gazeux à l'état solide) du dioxyde de carbone est provoquée. La température de désublimation peut être également désignée indifféremment de température de condensation solide, de cristallisation ou encore de sublimation inverse.

Avantageusement, l'agent de liquéfaction est injecté au niveau du reflux de méthane.

Avantageusement, l'agent de liquéfaction est injecté dans la première colonne de distillation à une température T1 de l'ordre de -100°C.

Avantageusement, l'agent de liquéfaction est soumis à un refroidissement en deux étages sous pression avant son injection dans la tête de la première colonne de distillation pour atteindre la température T1.

Avantageusement, la tête de la première colonne de distillation est mise en froid en tout ou partie par l'agent de liquéfaction.

Avantageusement, le procédé comporte une étape de récupération du méthane à l'état gazeux à l'issue de la première séparation.

Selon un l'invention, il est procédé à une deuxième séparation des composés du mélange liquide récupéré à l'issue de la première séparation et composé du dioxyde de carbone, de l'agent de liquéfaction et des éventuels hydrocarbures. Cette deuxième séparation est réalisée par distillation cryogénique dans une deuxième colonne de distillation après détente du mélange liquide pour atteindre une température et une pression d'équilibre permettant la séparation du dioxyde de carbone sous forme de condensat en tête de colonne et l'agent de liquéfaction additionné des éventuels hydrocarbures en résidus sous forme liquide en bas de colonne.

Avantageusement, la deuxième séparation est réalisée par mise en froid de la tête de la deuxième colonne à la température de condensation du dioxyde de carbone comprise entre -50 ° Celsius et -60° Celsius en fonction de la pression atteinte après la détente.

Avantageusement, le procédé de séparation comporte une étape de récupération du dioxyde de carbone à l'état gazeux (condensat) à l'issue de la deuxième séparation.

Avantageusement, le procédé comporte une étape de récupération de l'agent de liquéfaction additionné des éventuels hydrocarbures à l'issue de la deuxième séparation pour son réacheminement en tout ou partie vers la première colonne de distillation.

Avantageusement, l'agent de liquéfaction est un hydrocarbure linéaire ou non linéaire du type alcène de la famille C₃ à C₇ou un mélange d'hydrocarbures de la famille C₃ à C₇.

Avantageusement, l'agent de liquéfaction est un hydrocarbure ou un mélange d'hydrocarbures identique(s) ou de propriétés physico-chimiques équivalentes à l'éventuel hydrocarbure ou mélange d'hydrocarbures présent dans le biogaz.

L'invention est appliquée dans une installation permettant la séparation de tout ou partie d'un biogaz à l'état liquide ou à l'état diphasique liquide/vapeur, contenant du méthane, du dioxyde de carbone et éventuellement un hydrocarbure ou un mélange d'hydrocarbures, mettant en œuvre le procédé de séparation tel que décrit précédemment. L'installation comprend à cet effet une première colonne de distillation destinée à séparer le méthane des autres composés par distillation cryogénique, ladite première colonne de distillation comprenant une alimentation principale pour l'injection du biogaz liquéfié à une température d'équilibre permettant l'obtention d'un mélange diphasique assurant la séparation des différents composés et une alimentation secondaire pour l'injection d'un agent de liquéfaction à l'état liquide composé d'un mélange d'hydrocarbure(s) de la famille C3 à C7, l'alimentation secondaire étant arrangée pour injecter l'agent de liquéfaction en tête de la première colonne de distillation, au-dessus du niveau d'entrée du biogaz. Selon un mode de réalisation avantageux, il peut être prévu que l'installation comprenne également une deuxième colonne de distillation pour la séparation des composés du mélange liquide récupéré à l'issue de la première séparation et composé du dioxyde de carbone, de l'agent de liquéfaction et des éventuels hydrocarbures.

L'avantage du procédé de séparation selon l'invention est de permettre d'obtenir des taux de pureté élevés des composés séparés du biogaz, soit de l'ordre de 99,9 % pour chacun des composés (méthane et dioxyde de carbone) et du même ordre pour l'agent de liquéfaction récupéré en sortie de la deuxième séparation.

L'avantage du procédé de séparation selon l'invention, dans le cas d'un biogaz non liquide ou bien à l'état diphasique, est d'empêcher la formation de neige carbonique en tête de colonne lors de la séparation du méthane des autres composés du biogaz.

### BREVE DESCRIPTION DES FIGURES

D'autres caractéristiques et avantages de l'invention ressortiront de la description détaillée de l'invention qui va suivre en référence aux figures annexées et dans lesquelles :
[Fig.1] La [Fig.1] représente une vue schématique d'un exemple de réalisation d'une installation pour la séparation de tout ou partie des composés d'un biogaz à l'état liquide ou dans un état diphasique liquide/vapeur mettant en œuvre un procédé de séparation selon l'invention ;
[Fig.2] La [Fig.2] représente les principales étapes d'un procédé de séparation de tout ou partie des composés d'un biogaz à l'état liquide ou dans un état diphasique liquide/ vapeur selon l'invention.

### DESCRIPTION DETAILLEE DE L'INVENTION

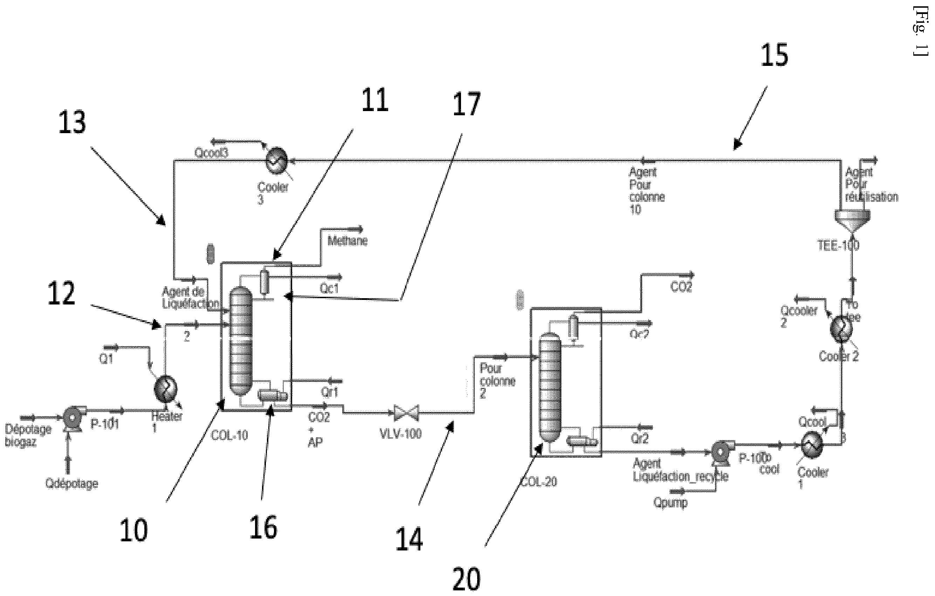

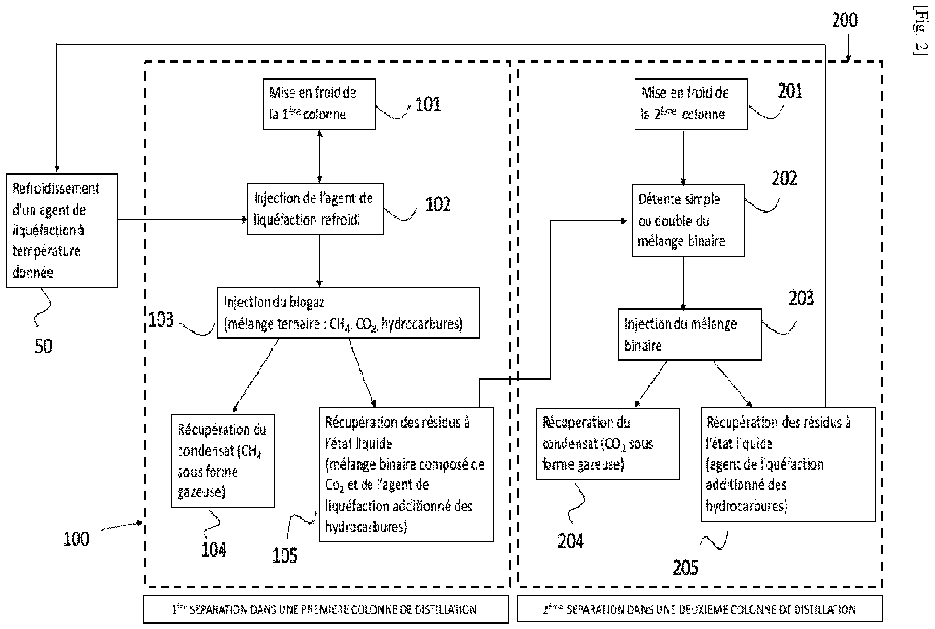

En relation avec figures 1 et 2, il est décrit un procédé de séparation des composés d'un biogaz à l'état liquide ou dans un état diphasique liquide/vapeur et l'installation prévue à cet effet.

Dans l'exemple décrit, le biogaz à traiter contient du méthane, du dioxyde de carbone ainsi qu'un hydrocarbure ou un mélange d'hydrocarbures de la famille C₃ à C₇, l'hydrocarbure ou le mélange d'hydrocarbures ayant été introduit pour assurer la liquéfaction préalable en tout ou partie du biogaz. Le biogaz liquéfié sera également désigné « mélange ternaire » dans la présente demande, étant composé majoritairement de méthane, de dioxyde de carbone et d'un hydrocarbure. Dans l'exemple décrit ci-après, il est considéré un biogaz liquéfié ou rendu majoritairement liquide par du n-butane (C₄). L'invention n'étant bien entendu pas limitée à cet hydrocarbure, la description qui suit reste transposable à un biogaz liquéfié ou rendu majoritairement liquide par un hydrocarbure ou un mélange d'hydrocarbures de la famille C₃ à C₇ autre que C₄.

La séparation des composés du biogaz est basée sur une double distillation. L'installation 1, illustrée sur la [Fig.1], comprend ainsi une première colonne de distillation 10 destinée à épurer le biogaz et une deuxième colonne de distillation 20 permettant de séparer le dioxyde de carbone des autres composés.

La première colonne de distillation 10 comporte des plateaux théoriques définissant les étages de la colonne, un garnissage pour favoriser le barbotage ainsi qu'une surface d'échange adéquats aux vapeurs montantes au travers de liquides descendants, un condenseur 11 raccordé fluidiquement à le première colonne de distillation 10, au sommet de cette dernière, ainsi que des moyens de chauffage, du type bouilleur, ménagés au pied de la première colonne.

La première colonne de distillation 10 comporte en outre une double alimentation : une première alimentation, désignée alimentation principale 12, pour l'injection du biogaz et une deuxième alimentation, désignée alimentation secondaire 13, pour l'injection d'un agent de liquéfaction à l'état liquide. Ce dernier sera décrit en détail plus loin. L'alimentation principale 12 délimite la partie haute de la colonne (tête de colonne) de la partie basse de la colonne. L'alimentation secondaire 13 est arrangée pour permettre l'injection de l'agent de liquéfaction en tête de colonne, avantageusement au premier étage théorique de ladite colonne, mais de manière préférée au niveau du reflux 17 du condenseur.

La deuxième colonne de distillation 20 comprend un arrangement similaire à celui de la première colonne si ce n'est qu'elle ne comporte qu'une seule alimentation pour injecter les résidus issus de l'opération de séparation dans la première colonne de distillation 10 et acheminés via un circuit 14 présentant comme point d'entrée un réservoir 16 dans lequel les résidus ont été stockés après récupération au fond de la première colonne de distillation 10 et comme point de sortie, la deuxième colonne de distillation 20. Elle comporte ainsi des plateaux définissant les étages théoriques de la colonne, un garnissage pour favoriser le barbotage adéquat de vapeurs montantes au travers de liquides descendants, un condenseur raccordé fluidiquement à la deuxième colonne de distillation 20, au sommet de cette dernière, ainsi que des moyens de chauffage, du type bouilleur, ménagés au pied de la première colonne de distillation 10.

L'installation 1 comporte en outre un circuit de réacheminement 15 de l'agent de liquéfaction additionné du ou des hydrocarbure(s) initialement présent(s) dans le biogaz vers la première colonne de distillation 10, le circuit de réacheminement 15 comprenant comme point d'entrée le fond de la deuxième colonne de distillation 20 et comme point de sortie, l'alimentation secondaire 13 de la première colonne.

Selon le procédé de séparation ([Fig.2]), le biogaz est soumis à une première séparation (étape 100) pour séparer le méthane des autres composés (dioxyde de carbone et hydrocarbures). Cette première séparation est réalisée par distillation cryogénique dans la première colonne de distillation 10.

Plus particulièrement, la première séparation est réalisée en injectant dans la première colonne de distillation 10, en alimentation principale, le biogaz liquéfié à une température d'équilibre permettant d'obtenir un mélange diphasique permettant la séparation des différents composés (étape 103).

Préalablement à son introduction dans la première colonne de distillation 10, le mélange ternaire est chauffé jusqu'à une température comprise entre -60°C et -50°C , la pression étant de l'ordre de 21 bars. Le mélange ainsi chauffé est introduit dans la première colonne de distillation 10 pour séparer le méthane du dioxyde de carbone et des hydrocarbures. Le méthane constituant le composé le plus léger par rapport au mélange d'hydrocarbures et de dioxyde de carbone s'élève sous forme gazeuse en tête de colonne tandis que le dioxyde de carbone et les hydrocarbures descendent sous forme liquide en bas de la colonne. Le méthane forme le condensat en tête de la première colonne tandis que le mélange d'hydrocarbures et de dioxyde de carbone forme les résidus en bas de colonne.

Afin d'éviter le givrage du dioxyde de carbone, un agent de liquéfaction est injecté via l'alimentation secondaire 13 à une température T1 inférieure ou égale à la température qui provoquerait une désublimation du dioxyde de carbone dans les conditions opératoires de la colonne de distillation, en quantité proportionnelle au débit de vapeur du dioxyde de carbone ascendant en tête de colonne (étape 102). Avantageusement, l'agent de liquéfaction injecté est composé d'un hydrocarbure ou d'un mélange d'hydrocarbure(s) de la famille C₃ à C₇sous forme liquide. De manière préférée, il s'agit d'un hydrocarbure linéaire ou non linéaire (type alcène) ou un mélange d'hydrocarbures de la famille C₃ à C₇. Dans l'exemple décrit, l'agent de liquéfaction choisi est identique à l'hydrocarbure présent dans le biogaz à épurer et qui avait été utilisé pour liquéfier le biogaz, à savoir du n-butane (C4). Dans ce cas, la température T1 à laquelle l'agent de liquéfaction est injecté sera de l'ordre de -100°C.

De manière avantageuse, l'agent de liquéfaction est injecté au même niveau que le reflux du condenseur.

Avantageusement, l'agent de liquéfaction est soumis à un refroidissement sous pression avant son injection dans la tête de la première colonne de distillation 10 pour atteindre la température T1 (étape 50). Dans l'exemple décrit, il est ainsi porté de la température ambiante à la température de -100°C en passant par deux étages de refroidissement via des échangeurs, un premier étage à -56°C suivi d'un deuxième étage à - 100°C.

Aux fins de procéder à la première séparation, la tête de colonne est préalablement refroidie pour être ainsi portée à la température de condensation du méthane (étape 101). Dans l'exemple décrit, la tête de colonne est avantageusement portée à la température de -106 °C pour une pression de l'ordre de 20 bars. Avantageusement, la tête de la première colonne de distillation 10 est mise en froid aussi par l'agent de liquéfaction (cette mise en froid est représentée sur la [Fig.2] par la double flèche entre l'étape 101 et l'étape 102). Ce dernier, refroidi préalablement (étape 50), est injecté à la colonne à la température de -100°C avec un débit suffisant pour générer un mélange ternaire avec le méthane, le dioxyde de carbone et le butane initialement présent.

Une fois que la colonne a atteint l'équilibre, le dépotage du mélange ternaire commence à l'aide d'une pompe. Le mélange est ensuite chauffé à -56°C. Une fois séparé du reste des composés, le méthane est récupéré (étape 104) tandis que le résidu composé du dioxyde de carbone et de l'agent de liquéfaction additionné de l'hydrocarbure initialement présent est détendu après passage par le bouilleur et poursuit la deuxième phase de distillation dans la deuxième colonne de distillation.

Dans l'exemple décrit, il est procédé à la séparation du dioxyde de carbone. Pour ce faire, le résidu composé du dioxyde de carbone et du mélange d'hydrocarbures, en l'espèce un mélange de butane (mélange composé de l'hydrocarbure présent dans le biogaz et de l'agent de liquéfaction injecté en tête de la colonne 10) est soumis à une deuxième distillation cryogénique dans la deuxième colonne de distillation 20 (étape 200).

De même, une mise en froid préalable de la tête de la deuxième colonne est nécessaire avant le lancement de la distillation (étape 201). Dans l'exemple décrit, elle est mise à la température de condensation du dioxyde de carbone comprise entre -50 ° Celsius et -60° Celsius en fonction de la pression atteinte après la détente (détente jusqu'à 5,5 bars). Une fois la deuxième colonne ayant atteint son point d'équilibre, le mélange binaire est introduit dans la deuxième colonne de distillation (étape 203) après avoir été soumis au préalable à une détente (jusqu'à 5,5 bars) (étape 202) pour atteindre une température et une pression d'équilibre permettant la séparation du dioxyde de carbone sous forme de condensat en tête de colonne et l'agent de liquéfaction additionné d'éventuels mélanges d'hydrocarbures en résidus sous forme liquide en bas de colonne. Lors de l'injection du mélange binaire dans la deuxième colonne de distillation 20, le dioxyde de carbone subit une vaporisation et s'élève en tête de la deuxième colonne à l'état gazeux tandis que le mélange d'hydrocarbures descend à l'état liquide en bas de la deuxième colonne de distillation 20.

Une fois séparé du reste des composés, le dioxyde de carbone est récupéré (étape 204) tandis que le mélange d'hydrocarbures est récupéré en pied de la colonne de distillation pour être éventuellement réacheminé en tout ou partie vers la première colonne de distillation 10 ou un réservoir de stockage tampon pour éventuellement être réutilisé pour liquéfier un biogaz (étape 205).

Dans le mode de réalisation précédemment décrit, il est procédé à la séparation de l'ensemble des composés constituant le biogaz. Il peut être prévu cependant qu'une partie des composés seulement soit séparée pour récupérer seulement une partie du biogaz, en l'espèce le méthane.

De même, dans le mode de réalisation précédemment décrit, le biogaz liquéfié soumis au procédé de séparation selon l'invention est un mélange ternaire composé majoritairement de méthane, de dioxyde de carbone et d'un hydrocarbure de la famille C₃ à C₇. Il peut être prévu cependant que le biogaz composé majoritairement de méthane et de dioxyde de carbone, le procédé de séparation et l'installation pouvant être mis en œuvre pour épurer un tel biogaz sans sortir du cadre de l'invention. De même, le biogaz en alimentation de la colonne est un mélange à l'état liquide ou bien à l'état diphasique liquide/vapeur.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Procédé de séparation de tout ou partie de composés d'un biogaz à l'état liquide ou à l'état diphasique liquide/vapeur, contenant du méthane, du dioxyde de carbone et éventuellement d'un hydrocarbure ou d'un mélange d'hydrocarbures de la famille C3 à C7, **caractérisé en ce qu'**une première séparation pour séparer le méthane des autres composés est réalisée par distillation cryogénique dans une première colonne de distillation (10) comportant une tête de colonne portée à la température de condensation du méthane à une pression donnée, la première séparation étant réalisée en injectant dans la première colonne de distillation (10) :
- en alimentation principale, le biogaz liquéfié à une température d'équilibre permettant l'obtention d'un mélange diphasique assurant la séparation des différents composés,
- en alimentation secondaire, un agent de liquéfaction à l'état liquide composé d'un mélange d'hydrocarbure(s) de la famille C3 à C7, l'agent de liquéfaction étant injecté en tête de la première colonne de distillation (10), au-dessus du niveau d'entrée du biogaz, à une température T1 inférieure ou égale à la température de désublimation du dioxyde de carbone à une pression donnée de la colonne de distillation et en quantité proportionnelle au débit de vapeur du dioxyde de carbone ascendant en tête de colonne,
et **en ce qu'**une deuxième séparation des composés du mélange liquide récupéré à l'issue de la première séparation et composé du dioxyde de carbone, de l'agent de liquéfaction et des éventuels hydrocarbures est réalisée par distillation cryogénique dans une deuxième colonne de distillation (20) après détente du mélange liquide pour atteindre une température et une pression d'équilibre permettant la séparation du dioxyde de carbone sous forme de condensat en tête de colonne et l'agent de liquéfaction additionné des éventuels hydrocarbures en résidus sous forme liquide en bas de colonne.

2. Procédé de séparation selon la revendication 1, **caractérisé en ce que** l'agent de liquéfaction est injecté au niveau du reflux de méthane.

3. Procédé de séparation selon la revendication 1 ou la revendication 2, **caractérisé en ce que** l'agent de liquéfaction est injecté dans la première colonne de distillation (10) à une température T1 de l'ordre de -100°C.

4. Procédé de séparation selon l'une quelconque des revendications précédentes, **caractérisé en ce que** l'agent de liquéfaction est soumis à un refroidissement en deux étages sous pression avant son injection dans la tête de la première colonne de distillation (10) pour atteindre la température T1.

5. Procédé de séparation selon l'une quelconque des revendications précédentes, **caractérisé en ce que** la tête de la première colonne de distillation (10) est mise en froid en tout ou partie par l'agent de liquéfaction.

6. Procédé de séparation selon l'une quelconque des revendications précédentes, **caractérisé en ce qu'**il comporte une étape de récupération du méthane à l'état gazeux à l'issue de la première séparation.

7. Procédé de séparation selon l'une quelconque des revendications précédentes, **caractérisé en ce que** la deuxième séparation est réalisée par mise en froid de la tête de la deuxième colonne de distillation (20) à la température de condensation du dioxyde de carbone comprise entre -50 ° Celsius et -60° Celsius en fonction de la pression atteinte après la détente.

8. Procédé de séparation selon l'une quelconque des revendications précédentes, **caractérisé en ce qu'**il comporte une étape de récupération du dioxyde de carbone à l'état gazeux à l'issue de la deuxième séparation.

9. Procédé de séparation selon l'une quelconque des revendications précédentes, **caractérisé en ce qu'**il comporte une étape de récupération de l'agent de liquéfaction additionné des éventuels hydrocarbures à l'issue de la deuxième séparation pour son réacheminement en tout ou partie vers la première colonne de distillation (10).

10. Procédé de séparation selon l'une quelconque des revendications précédentes, **caractérisé en ce que** l'agent de liquéfaction est un hydrocarbure linéaire ou non linéaire du type alcène de la famille C3 à C7 ou un mélange d'hydrocarbures de la famille C3 à C7.

11. Procédé de séparation selon l'une quelconque des revendications précédentes, **caractérisé en ce que** l'agent de liquéfaction est un hydrocarbure ou un mélange d'hydrocarbures identique(s) ou de propriétés physico-chimiques équivalentes à l'éventuel hydrocarbure ou mélange d'hydrocarbures présent dans le biogaz.

## Patentansprüche

1. Verfahren zur Trennung von Verbindungen eines Biogases, ganz oder teilweise, im flüssigen oder zweiphasigen flüssigen/dampfförmigen Zustand, das Methan, Kohlendioxid und gegebenenfalls einen Kohlenwasserstoff oder ein Gemisch aus Kohlenwasserstoffen der Familie C3 bis C7 enthält, **dadurch gekennzeichnet, dass** eine erste Trennung zur Trennung des Methans von den anderen Verbindungen durch kryogene Destillation in einer ersten Destillationssäule (10) ausgeführt wird, die einen Säulenkopf aufweist, der auf die Verflüssigungstemperatur des Methans bei einem bestimmten Druck gebracht wird, wobei die erste Trennung durch Einspritzen von Folgendem in die erste Destillationssäule (10) ausgeführt wird:
- als Hauptversorgung, das bei einer Gleichgewichtstemperatur verflüssigte Biogas, das ein zweiphasiges Gemisch ermöglicht, das die Trennung der verschiedenen Verbindungen gewährleistet,
- als Sekundärversorgung, ein Verflüssigungsmittel im flüssigen Zustand, das aus einem Gemisch aus Kohlenwasserstoff(en) der Familie C3 bis C7 besteht, wobei das Verflüssigungsmittel an dem Kopf der ersten Destillationssäule (10), oberhalb des Eintrittsniveaus des Biogases, bei einer Temperatur T1 kleiner oder gleich der Desublimationstemperatur des Kohlendioxids bei einem bestimmten Druck der Destillationssäule und in einer Menge proportional zu dem Dampfdurchsatz des an dem Säulenkopf aufsteigenden Kohlendioxids eingespritzt wird, und dadurch, dass eine zweite Trennung der Verbindungen aus dem nach der ersten Trennung zurückgewonnenen flüssigen Gemisch aus Kohlendioxid besteht, des Verflüssigungsmittels und eventueller Kohlenwasserstoffe durch kryogene Destillation in einer zweiten Destillationssäule (20) nach Ausdehnung des flüssigen Gemischs ausgeführt wird, um eine Temperatur und einen Gleichgewichtsdruck zu erreichen, die die Trennung des Kohlendioxids in Form von Kondensat an dem Säulenkopf und des Verflüssigungsmittels zusammen mit eventuellen Kohlenwasserstoffen als Rückstände in flüssiger Form an dem Boden der Säule ermöglichen.

2. Trennverfahren nach Anspruch 1, **dadurch gekennzeichnet, dass** das Verflüssigungsmittel an dem Methanrückfluss eingespritzt wird.

3. Trennverfahren nach Anspruch 1 oder Anspruch 2, **dadurch gekennzeichnet, dass** das Verflüssigungsmittel bei einer Temperatur T1 im Bereich von -100 °C in die erste Destillationssäule (10) eingespritzt wird.

4. Trennverfahren nach einem der vorhergehenden Ansprüche, **dadurch gekennzeichnet, dass** das Verflüssigungsmittel einer zweistufigen Druckkühlung unterzogen wird, bevor es in den Kopf der ersten Destillationssäule (10) eingespritzt wird, um die Temperatur T1 zu erreichen.

5. Trennverfahren nach einem der vorhergehenden Ansprüche, **dadurch gekennzeichnet, dass** der Kopf der ersten Destillationssäule (10) ganz oder teilweise durch das Verflüssigungsmittel abgekühlt wird.

6. Trennverfahren nach einem der vorhergehenden Ansprüche, **dadurch gekennzeichnet, dass** es einen Schritt zur Rückgewinnung des Methans im gasförmigen Zustand nach der ersten Trennung aufweist.

7. Trennverfahren nach einem der vorhergehenden Ansprüche, **dadurch gekennzeichnet, dass** die zweite Trennung durch Abkühlen des Kopfes der zweiten Destillationssäule (20) auf die Kondensationstemperatur des Kohlendioxids zwischen -50 Celsius und -60° Celsius in Abhängigkeit von dem nach der Ausdehnung erreichten Druck ausgeführt wird.

8. Trennverfahren nach einem der vorhergehenden Ansprüche, **dadurch gekennzeichnet, dass** es einen Schritt der Rückgewinnung des gasförmigen Kohlendioxids nach der zweiten Trennung aufweist.

9. Trennverfahren nach einem der vorhergehenden Ansprüche, **dadurch gekennzeichnet, dass** es einen Schritt zur Rückgewinnung des Verflüssigungsmittels zusammen mit eventuellen Kohlenstoffen nach Abschluss der zweiten Trennung aufweist, um es ganz oder teilweise zu der ersten Destillationssäule (10) zurückzuleiten.

10. Trennverfahren nach einem der vorhergehenden Ansprüche, **dadurch gekennzeichnet, dass** das Verflüssigungsmittel ein linearer oder nichtlinearer Kohlenwasserstoff des Alkentyps der Familie C₃ bis C₇ oder ein Gemisch aus Kohlenwasserstoffen der Familie C₃ bis C₇ ist.

11. Trennverfahren nach einem der vorhergehenden Ansprüche, **dadurch gekennzeichnet, dass** das Verflüssigungsmittel ein Kohlenwasserstoff oder ein Gemisch aus Kohlenwasserstoffen ist, der/die identisch ist/sind oder physikalischchemische Eigenschaften hat/haben, die dem eventuell in dem Biogas vorhandenen Kohlenwasserstoff oder dem Gemisch aus Kohlenwasserstoffen gleichwertig sind.

## Claims

1. Method for separating all or some of the compounds from a biogas in the liquid state or in the two-phase liquid/vapour state containing methane, carbon dioxide and optionally a hydrocarbon or a mixture of hydrocarbons from the C3 to C7 family, **characterised in that** a first separation for separating methane from the other compounds is performed by cryogenic distillation in a first distillation column (10) comprising a column top brought to the condensation temperature of methane at a given pressure, the first separation being performed by injecting into the first distillation column (10):
- as a main supply, the biogas liquefied to an equilibrium temperature which makes it possible to obtain a two-phase mixture, ensuring the separation of the different compounds,
- as a secondary supply, a liquefying agent, in the liquid state, composed of a mixture of hydrocarbon(s) from the C3 to C7 family, the liquefying agent being injected at the top of the first distillation column (10), above the biogas inlet, at a temperature T1 lower than or equal to the carbon dioxide desublimation temperature at a given pressure of the distillation column and in an amount proportional to the vapour flow rate of the carbon dioxide ascending at the top of the column,
and **in that** a second separation of the compounds of the liquid mixture recovered from the first separation and compound of the carbon dioxide, the liquefying agent and optional hydrocarbons is performed by cryogenic distillation in a second distillation column (20) after expansion of the liquid mixture to reach an equilibrium temperature and pressure enabling the separation of the carbon dioxide in the form of condensate at the top of the column and the liquefying agent added with optional hydrocarbons in residues in liquid form at the bottom of the column.

2. Separation method according to claim 1, **characterised in that** the liquefying agent is injected at the methane reflux.

3. Separation method according to claim 1 or claim 2, **characterised in that** the liquefying agent is injected into the first distillation column (10) at a temperature T1 of around -100°C.

4. Separation method according to any one of the preceding claims, **characterised in that** the liquefying agent is subjected to a pressurised, two-stage cooling before it is injected into the top of the first distillation column (10) to reach the temperature T1.

5. Separation method according to any one of the preceding claims, **characterised in that** the top of the first distillation column (10) is totally or partially cooled by the liquefying agent.

6. Separation method according to any one of the preceding claims, **characterised in that** it comprises a step of recovering methane in the gaseous state from the first separation.

7. Separation method according to any one of the preceding claims, **characterised in that** the second separation is performed by cooling the top of the second distillation column (20) to the condensation temperature of carbon dioxide, of between -50°C and -60°C depending on the pressure reached after expansion.

8. Separation method according to any one of the preceding claims, **characterised in that** it comprises a step of recovering carbon dioxide in the gaseous state from the second separation.

9. Separation method according to any one of the preceding claims, **characterised in that** it comprises a step of recovering the liquefying agent added with optional hydrocarbons from the second separation for it to be totally or partially rerouted to the first distillation column (10).

10. Separation method according to any one of the preceding claims, **characterised in that** the liquefying agent is a linear or non-linear hydrocarbon of the alkene type from the C₃ to C₇ family, or a mixture of hydrocarbons from the C₃ to C₇ family.

11. Separation method according to any one of the preceding claims, **characterised in that** the liquefying agent is a hydrocarbon or a mixture of identical hydrocarbons, or hydrocarbons of physico-chemical properties equivalent to the optional hydrocarbon or mixture of hydrocarbons present in the biogas.
